# EUROPÄISCHE PATENTSCHRIFT

(11) **EP 2 030 599 B1**
(45) Veröffentlichungstag und Bekanntmachung des Hinweises auf die Patenterteilung: **19.10.2011**
(21) Anmeldenummer: 07115423.1
(22) Anmeldetag: 31.08.2007
(51) Int. Cl.: A61F 9/01

(54) **Lasersystem zum Ablatieren von Hornhaut an einem Patientenauge**
Laser system for ablating the cornea in a patient's eye
Système laser pour l'ablation de cornée dans un oeil de patient

(43) Veröffentlichungstag der Anmeldung: 04.03.2009
(73) Patentinhaber: Schwind eye-tech-solutions GmbH & Co. KG, 63801 Kleinostheim (DE)
(72) Erfinder: Hollerbach, Thomas, 63808 Haibach-Dörrmorsbach (DE); Grimm, Anita, 63928 Eichenbühl (DE); Arba-Mosquera, Samuel, 63739 Aschaffenburg (DE)
(74) Vertreter: Laub, Alexander

(56) Entgegenhaltungen:
- EP-A- 0 280 414
- EP-A- 0 412 789
- WO-A-96/21407
- WO-A-96/22751
- US-A1- 2002 099 362
- US-A1- 2004 054 356

## Beschreibung

Die Erfindung betrifft ein Lasersystem zum Ablatieren von Hornhaut an einem Patientenauge. In einem solchen Lasersystem sendet im Betrieb eine Laserquelle einen Laserstrahl aus, der nach dem Durchlaufen eines optischen Systems zur Strahlformung und einer Zieleinrichtung, die den Laserstrahl gegebenenfalls ablenkt, das Lasersystem verlässt. Das Auge eines außerhalb des Lasersystems befindlichen Patienten wird von dem Laserstrahl getroffen. Die Zieleinrichtung wird hierbei von einer Steuereinheit angesteuert und lenkt in Abhängigkeit von Steuersignalen den Laserstrahl wahlweise auf unterschiedliche Stellen der Hornhaut des Patientenauges.

Ein Lasersystem zum Ablatieren von Hornhaut mit einer solchen Zieleinrichtung ist zu unterscheiden von in der Frühphase der Laserablation verwendeten Lasersystemen, bei denen ein Laserstrahl großflächig auf das gesamte Auge gestrahlt wurde. In diesem Zusammenhang ist es bekannt gewesen, den Laserstrahl auf bestimmte Weise zu formen, vergleiche zum Beispiel die EP 0 280 414 A1. Im Rahmen der Strahlformung kam es dazu, dass Anteile des Laserstrahls nicht auf das Patientenauge gelangten.

Bei dem Vorliegen des beanspruchten Lasersystems zum Ablatieren von Hornhaut an einem Patientenauge ist die Zieleinrichtung, die den Laserstrahl wahlweise auf unterschiedliche Stellen des Patientenauges lenkt, vorausgesetzt.

Grundsätzlich werden solche Zieleinrichtungen dann eingesetzt, wenn der Laserstrahl einen vergleichsweise geringen Fleck auf dem Patientenauge trifft. Zieleinrichtungen sind aber auch bekannt im Zusammenhang mit großflächigem Aufbringen eines Laserstrahls.

Es beschreibt beispielsweise die US 2004/0054356 A1, dass ein solcher großflächiger Strahl, der durch eine künstliche Iris geformt wird, mit Hilfe einer Linse zielgenau auf ein Patientenauge eingestellt werden kann. Die Linse bestimmt hierbei die Auftrefffläche.

Die US 2002/0099362 A1 beschreibt, dass Laserstrahlen nacheinander auf unterschiedliche Zellen des Patientenauges appliziert werden, dass aber die Fläche, auf der der Laserstrahl auftrifft, im Verhältnis zum gesamten zu ablatierenden Bereich relativ groß ist. Die Größe kann insbesondere variiert werden, wozu eine Blende eingesetzt wird.

Der Laserstrahl wird pulsweise ausgesandt. Jeder Puls trägt eine bestimmte Menge an Hornhaut ab, die unter anderem von der Intensität des Laserstrahls abhängig ist. Die Intensität des Laserstrahls in den einzelnen Pulsen bestimmt somit die Tiefenauflösung, die beim Abtragen eines Profils in der Hornhaut erreicht wird. Da die Intensität des von der Laserquelle ausgehenden Laserstrahls nicht kurzfristig von Puls zu Puls geändert werden kann, stehen zwei Erfordernisse zueinander in Widerspruch: Es gibt zum einen das Erfordernis, eine möglichst gute Auflösung zu erzielen, damit das in die Hornhaut eingeschriebene Profil möglichst glatt ist. Zum anderen soll aber die Gesamtdauer der Behandlung möglichst kurz sein. Je mehr man die Intensität verringert, um eine bessere Auflösung in der Tiefe beim Abtragen zu erreichen, desto länger wird die Behandlung jedoch.

Es ist Aufgabe der Erfindung, den Widerspruch aufzulösen und ein Lasersystem zum Ablatieren von Hornhaut an einem Patientenauge bereitzustellen, das bei nicht übermäßig langer Behandlungsdauer dennoch für eine gute Auflösung sorgt.

Die Aufgabe wird durch ein Lasersystem nach Patentanspruch 1 gelöst. Demgemäß umfasst das Lasersystem Auskoppelmittel, die der von der Laserquelle ausgesandte Laserstrahl vor dem Verlassen des Lasersystems durchläuft. Die Auskoppelmittel koppeln im Betrieb einen durch Wahl zwischen zumindest zwei Möglichkeiten festlegbaren (bzw. festgelegten) Teil (von zwischen einschließlich 0% und fast 100%) des Laserstrahls aus, so dass dieser Teil des Laserstrahls nicht das Lasersystem in Richtung Patientenauge, also über das Austrittsfenster, verlässt.

Durch das Verwenden gesonderter Auskoppelmittel zum Auskoppeln eines Teils des Laserstrahls beseitigt man die bei herkömmlichen Lasersystemen gegebene Zwangsbedingung, dass die Laserquelle allein die Energie der einzelnen Laserpulse bestimmt. Auskoppelmittel können insbesondere auch kurzfristig angesteuert werden. So ist es eine bevorzugte Ausführungsform, dass wenn die Laserquelle Laserpulse vorbestimmter Dauer mit einer vorbestimmten Frequenz abgibt, die Auskoppelmittel während der Pause zwischen zwei Laserpulsen umschaltbar sind (nämlich zwischen den beiden genannten Möglichkeiten). Es lässt sich somit kurzfristig der Anteil des ausgekoppelten Laserstrahls ändern. Individuellen Laserpulsen können individuelle Energien zugeordnet werden, und der Laserpuls mit der niedrigsten Energie bestimmt an dem Ort seines Auftreffens die Auflösung. Hingegen bestimmt die Anzahl der Laserpulse insgesamt die Dauer der Behandlung. Da die Laserpulse auch Laserpulse mit höherer Energie umfassen, ist die Dauer der Behandlung geringer, als wenn ausschließlich Laserpulse mit geringerer Energie auf der Hornhaut auftreffen würden.

Bei der Erfindung erfolgt die Wahl zwischen den bestehenden Möglichkeiten der Festlegung des ausgekoppelten Teils durch Ansteuerung der Auskoppelmittel von einer Steuereinheit. Es kann sich hierbei um dieselbe zentrale Steuereinheit handeln, die auch die Zieleinrichtung ansteuert und festlegt, welcher Laserstrahl wo auf der Hornhautoberfläche auftrifft. In dieser zentralen Steuereinheit ist üblicherweise auch das Ablationsprofil festgelegt, und die Steuereinheit kann zu einem Ort auf der Hornhaut dann festlegen, dass nach Aussenden einer bestimmten Anzahl von Laserpulsen höherer Energie auf eine Stelle der Hornhaut, durch die die Hauptabtragsarbeit geleistet wird, ein oder mehrere Laserpulse ausgesandt werden, die eine niedrigere Energie haben, um die Oberflächenformung zu verfeinern.

Bei der Erfindung umfasst das Lasersystem ferner eine den Auskoppelmitteln im Strahlengang nachgeordnete Messeinrichtung zur Messung der Energie und/oder der Energiedichte des Laserstrahls, und die Messergebnisse der Messeinrichtung werden der Steuereinheit zugeführt. Auf diese Weise ist eine Regelung ermöglicht, durch die die Genauigkeit bei der Ablation erhöht werden kann.

Bei einer einfach zu realisierenden Ausführungsform umfassen die Auskoppelmittel eine (verstellbare) Blende. Es gibt verschiedene Möglichkeiten, welche Art Blende verwendet werden kann.

Bei einer ersten Alternative bietet die Blende zumindest zwei Möglichkeiten von Blendenöffnungen, wobei sich die Blendenöffnungen durch ihre Größe unterscheiden. Die Größe bestimmt, welcher Teil des Laserstrahls ausgekoppelt wird. Man kann die Blende so zu dem eintreffenden Laserstrahl positionieren, dass genau die Mitte des Laserstrahls ausgekoppelt wird. Es hat sich jedoch als vorteilhaft erwiesen, wenn die Blende zu dem eintreffenden Laserstrahl so positioniert wird, dass die Blendenöffnung exzentrisch zum Laserstrahl ist. Dann ist der Effekt der unterschiedlichen Größe der Blende umso größer, d.h. der Unterschied in der Energie des das Lasersystem verlassenden (Rest-Laserstrahls) wird umso größer.

Bei einer zweiten Alternative bietet die Blende zumindest zwei Möglichkeiten von Blendenöffnungen, die sich durch ihr Zentrum voneinander unterscheiden. Hierbei wird davon ausgegangen, dass der Laserstrahl eine nicht gleichmäßige Energiedichte hat. Bei der einen Möglichkeit wird dann ein Teil des Laserstrahls mit eher höherer Energiedichte ausgekoppelt, und bei der anderen Möglichkeit ein Teil des Laserstrahls mit eher niedriger Energiedichte. Bei letzterer Alternative ist es die einfachste Ausführungsform, wenn die Blendenöffnungen die gleiche Größe haben, denn dann kann man die Blende so gestalten, dass die Blendenöffnung einfach versetzbar ist.

Alternativ zur Blende kann in den Auskoppelmitteln ein Strahlteiler vorgesehen werden, der den eintreffenden Laserstrahl in zwei Teilstrahlen aufteilt, von denen nur einer das Lasersystem verlässt. Was mit dem anderen Teilstrahl, der das Lasersystem nicht verlässt, geschieht, ist hierbei unwesentlich. Es kann vorgesehen sein, dass der Strahlteiler fakultativ in den Strahlengang eingesetzt wird, d.h. dass einmal ein Teil von 0% des Laserstrahls ausgekoppelt wird (Strahlteiler nicht eingekoppelt) und ein andermal ein von 0% verschiedener Teil des Laserstrahls. Bevorzugt ist der Strahlteiler jedoch immer eingekoppelt und bietet zumindest zwei Möglichkeiten, welche Energieanteile die Teilstrahlen relativ zum eintreffenden Laserstrahl haben.

Bei einer Ausführungsform ist der Strahlteiler ein halbdurchlässiger Spiegel, der dadurch die Energieanteile festlegt, dass er in einen vorbestimmten Winkel verkippt wird. Diese Ausführungsform ist deswegen vorteilhaft, weil den verkippbaren, halbdurchlässigen Spiegel ein Teilstrahl geradlinig durchläuft, so dass am Grundaufbau des Lasersystems im Vergleich zu herkömmlichen Lasersystemen wenig geändert werden muss. Bei einer alternativen Ausführungsform umfasst der Strahlteiler ein optisches Prisma, dessen Position bevorzugt variabel ist.

Nachfolgend werden bevorzugte Ausführungsformen der Erfindung unter Bezug auf die Zeichnung beschrieben, in der:

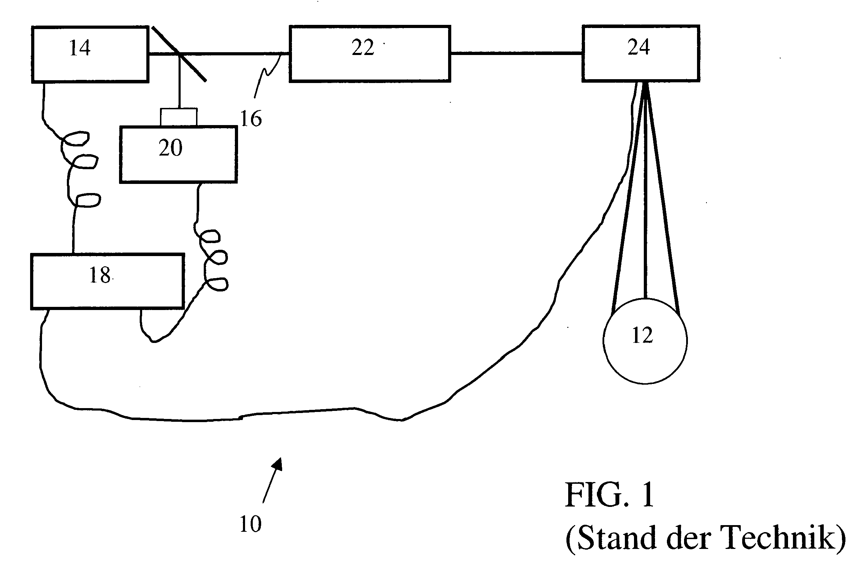

- Fig. 1: schematisch den Aufbau eines Lasersystems gemäß dem Stand der Technik veranschaulicht,
- Fig. 2: schematisch den Aufbau eines erfindungsgemäßen Lasersystems veranschaulicht,
- Fig. 3a und 3b: die Verwendung einer Blende als Auskoppelmittel bei einer ersten Ausführungsform in zwei verschiedenen Stellungen veranschaulichen,
- Fig. 4a und 4b: die Verwendung einer Blende als Auskoppelmittel bei einer zweiten Ausführungsform in zwei verschiedenen Stellungen veranschaulichen,
- Fig. 5a und 5b: die Verwendung eines halbdurchlässigen Spiegels als Auskoppelmittel bei zwei verschiedenen Stellungen veranschaulichen,
- Fig. 6a und 6b: die Verwendung eines optischen Prismas als Auskoppelmittel bei zwei verschiedenen Stellungen veranschaulichen.

Ein im Ganzen mit 10 bezeichnetes Lasersystem des Standes der Technik dient zum Abtragen (Ablatieren) von Hornhaut von einem schematisch dargestellten Patientenauge 12. Eine Laserquelle 14 erzeugt eine Folge von Laserpulsen. In der Figur ist der Laserstrahl mit 16 bezeichnet. Die Laserquelle 14 wird von einer Steuereinheit 18 angesteuert. Ein Energiesensor 20 misst die von der Laserquelle 14 abgegebene Energie und gibt das Messergebnis an die Steuereinheit 18 weiter, welche die Laserquelle 14 entsprechend ansteuern kann. Der Laserstrahl 16 wird durch ein optisches System 22 durch Strahlformung so geformt, dass sich ein gewünschtes Laserprofil ergibt. Der so geformte Laserstrahl wird einer Zieleinrichtung 24 zugeführt, die den Laserstrahl wahlweise auf unterschiedliche Stellen der Hornhaut des Patientenauges 12 lenkt. Die Zieleinrichtung 24 wird von der Steuereinheit 18 angesteuert, die auf diese Weise die Anzahl der Laserpulse zu unterschiedlichen Stellen auf der Hornhaut festlegt, um so das Abtragen eines vorbestimmten Ablationsprofils zu bewirken.

Die Energie des Laserstrahls 16 hängt von einer Einstellung der Laserquelle 14 ab, die nicht kurzfristig, also zwischen zwei Pulsen, die mit einer Frequenz von 100 - 1000 Hertz abgegeben werden, umgestellt werden kann. Es muss nun bisher mühsam ein Kompromiss gefunden werden zwischen der Abgabe von Laserstrahlen mit eher niedriger Energie zur Erhöhung der Auflösung der Abtragung und einer langen Behandlungsdauer einerseits und der Abgabe von Laserstrahlen mit eher höherer Energie unter Inkaufnahme einer schlechteren Auflösung bei der Abtragung, aber mit einer geringeren Behandlungsdauer, andererseits.

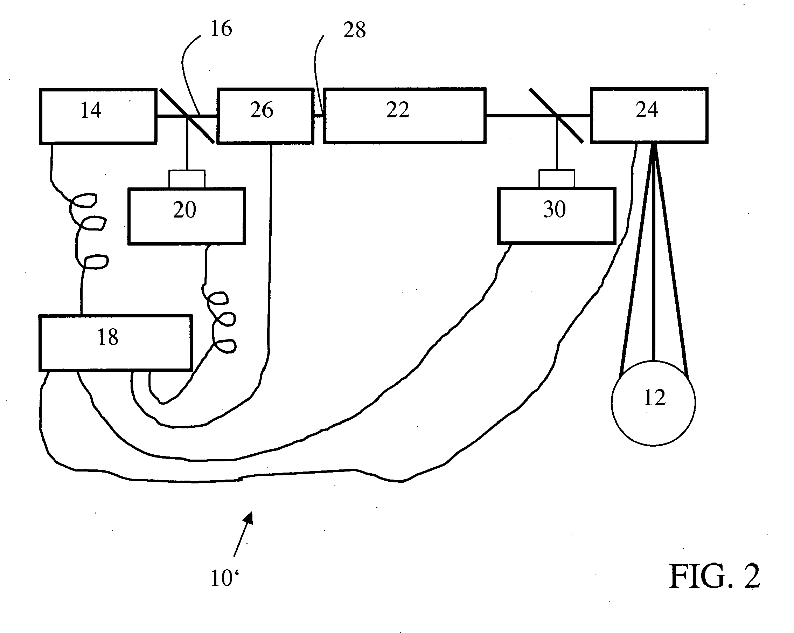

Ein erfindungsgemäßes in Fig. 2 gezeigtes Lasersystem 10' überwindet diese Zwänge: Das Lasersystem 10' unterscheidet sich von dem Lasersystem 10 dadurch, dass es (bevorzugt im Strahlengang vor dem optischen System 22 zur Strahlformung angeordnete) Mittel 26 zum Auskoppeln eines Teils des Laserstrahls umfasst: Von dem zu den Mitteln 26 gelangenden Laserstrahl 16 gelangt nur ein Anteil 28 in den weiteren Strahlengang weiter, vorliegend zu dem optischen System 22 zur Strahlformung. Die Auskoppelmittel 26 sind elektrisch über die Steuereinheit 18 ansteuerbar. Es ist ein weiterer elektrischer Sensor 30 im Strahlengang hinter den Auskoppelmittel 26 vorgesehen, der den Energiegehalt der Laserpulse 28 misst, wobei das Messergebnis der Steuereinheit 18 zugeführt wird und gegebenenfalls zur Regelung der Auskoppelmittel 26 verwendet wird.

Die Auskoppelmittel 26 erlauben es, zwischen zumindest zwei Möglichkeiten und bevorzugt einer Vielzahl von Möglichkeiten zu wählen, welcher Anteil des Laserstrahls 16 ausgekoppelt und als Laserstrahl 28 weitergeführt wird.

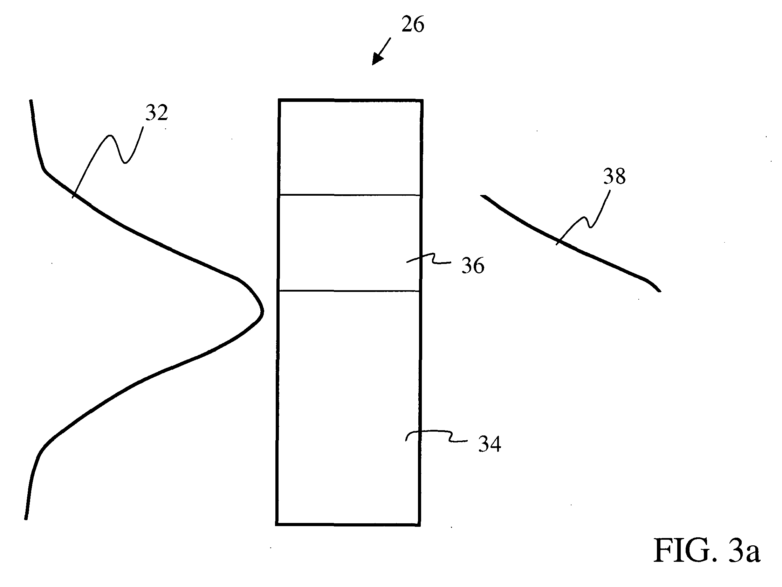

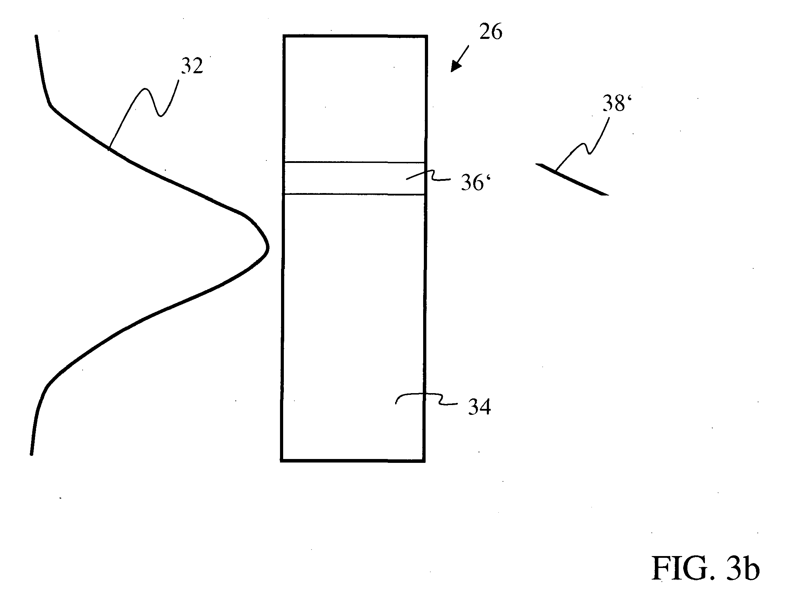

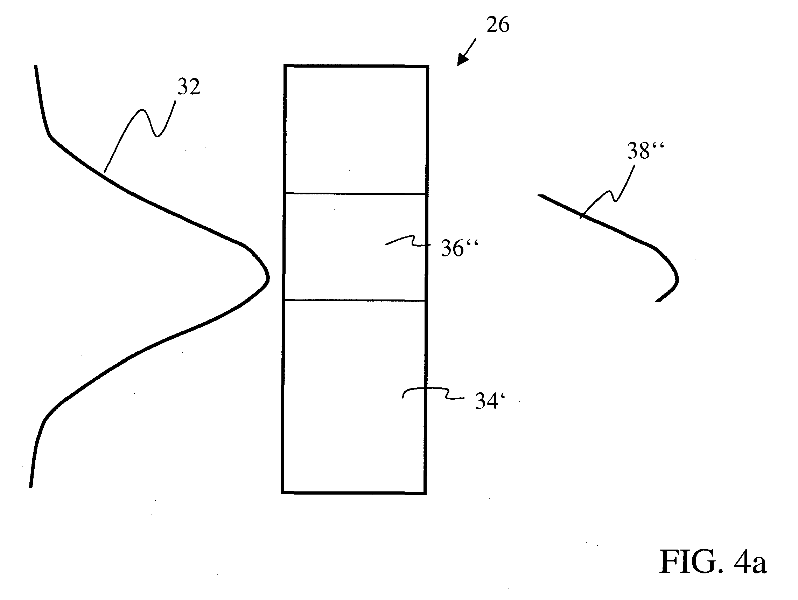

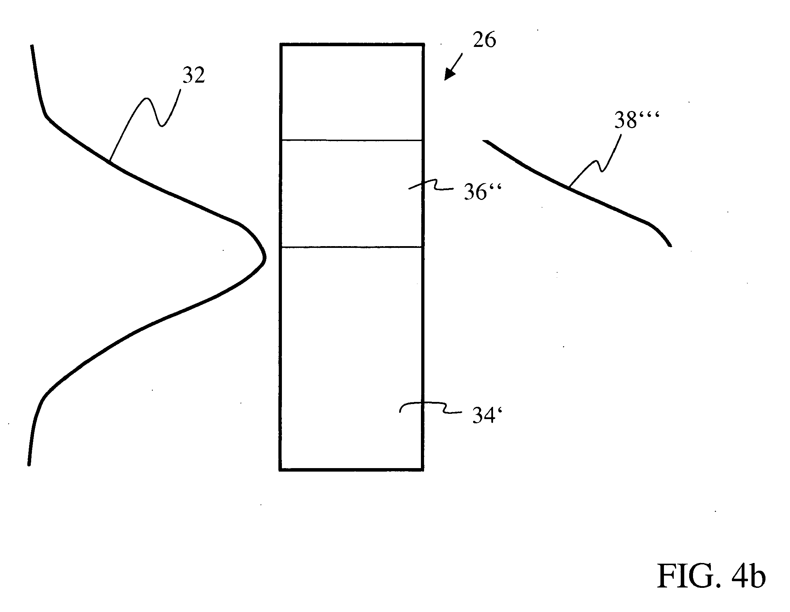

Bei einer Ausführungsform sind die Mittel 26 zum Auskoppeln durch eine Blende gestaltet. Zwar kann vorgesehen sein, dass die Blende einfach in einem Zustand den gesamten Laserstrahl 16 hindurch lässt und im anderen Zustand einen Teil auskoppelt, bei bevorzugten Ausführungsformen ist die Lage jedoch komplexer. Es gibt zwei bevorzugte Ausführungsformen von Blenden 34 und 34', die im Folgenden unter Bezug auf die Fig. 3a/3b und Fig. 4a/4b beschrieben werden.

Bei der ersten Ausführungsform umfassen die Mittel 26 zum Auskoppeln die Blende 34, zu der zwei unterschiedliche Blendenöffnungen 36 und 36' definiert sind, die sich durch ihre Größe unterscheiden. Bevorzugt ist die Blende 34 so angeordnet, dass die Öffnungen 36 und 36' exzentrisch zum Profil 32 eines eintreffenden Laserstrahls 16 sind. Dadurch wird der Effekt der unterschiedlichen Größe der Blendenöffnungen 36 und 36' verstärkt. Die Blendenöffnung 36 lässt den Anteil 38 des Strahlprofils 32 durch, die Blende 36' den Anteil 38'. Die Strahlprofilanteile 38 und 38' werden als Laserstrahl 28 dem optischen System 22 zur Strahlformung zugeleitet, und es wird ein Laserstrahl gebildet, der sich dann über eine vorbestimmte Pulsbreite erstreckt. Das Ausschneiden eines Anteils des Laserstrahlprofils 32 beeinträchtigt daher nicht die Gesamtfläche des endgültig auf die Hornhaut auftreffenden Laserstrahls und beeinflusst somit nicht die seitliche Auflösung. Vielmehr wird die Auflösung in der Tiefe beeinflusst.

So kann vorgesehen sein, dass zur Abtragung einer vorbestimmten Ablationstiefe z.B. zehn Laserpulse lang die Blendenöffnung 36 aufrecht erhalten wird, um hier viel Hornhaut abzutragen, und dass dann ein Laserpuls, eventuell auch mehrere Laserpulse lang die Blendenöffnung 36' eingestellt wird, damit die Ablationstiefe noch genauer eingestellt werden kann, ohne dass viel Hornhaut pro Puls abgetragen wird. Bei der Ausführungsform gemäß Fig. 4a/4b wird als Mittel 26 zum Auskoppeln eine Blende 34' verwendet, die eine Blendenöffnung 36" fester Größe hat, wobei die Blendenöffnung 36" jedoch in der Blende 34' zu unterschiedlichen Stellen wandern kann. Im Fall der Einstellung der Blendenöffnung 36", wie es in Fig. 4a gezeigt ist, lässt die Blende 34' von dem Laserstrahlprofil 32 den Anteil 38" durch. Verschiebt man die Blendenöffnung 36' ein bisschen zum Exzentrischen hin, lässt die Blendenöffnung 36" den Anteil 38"' des Laserstrahlprofils 32 durch. Da das Laserstrahlprofil 32 zum Exzentrischen hin abfällt, ist der Energiegehalt des Laserstrahlanteils 38"' kleiner als der des Laserstrahlanteils 38". Auch hier kann der stärkere Anteil 38" dann eingesetzt werden, wenn es um das Abtragen größerer Hornhautmengen geht, und bei der Feinabstimmung wird dann die Einstellung gemäß Fig. 4b vorgenommen, und der Anteil 38"' wird verwendet, um eine wohldefinierte Menge abzutragen. Durch das Vorhandensein des Energiesensors 30 und dessen Verbindung mit der Steuereinheit 18 kann das System geregelt arbeiten.

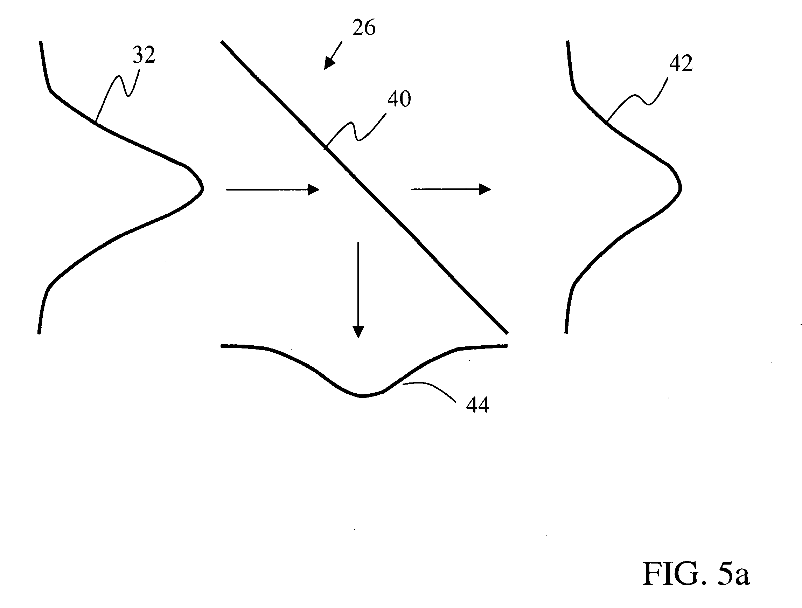

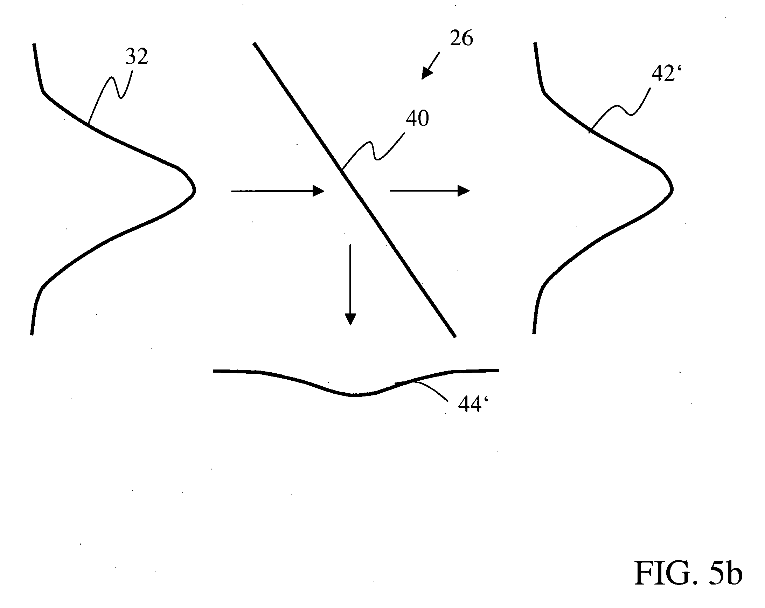

Bei der in den Fig. 5a und 5b veranschaulichten Ausführungsform umfassen die Mittel 26 zum Auskoppeln einen halbdurchlässigen Spiegel 40. Der halbdurchlässige Spiegel 40 ist verkippbar. Bei der in Fig. 5a gezeigten Stellung lässt er von dem Laserstrahlenprofil 32 den Strahlprofilanteil 42 geradlinig durch und reflektiert den Strahlprofilanteil 44 zur Seite hin. Wird der halbdurchlässige Spiegel 40 etwas steiler verkippt, wie in Fig. 5b dargestellt, lässt er den Strahlprofilanteil 42' durch, der einen höheren Energiegehalt hat als der Strahlprofilanteil 42, und gleichzeitig wird naturgemäß der Energiegehalt in dem reflektierten Strahlprofilanteil 44' im Vergleich zu dem Strahlprofilanteil 44 verringert. Auch hier gibt es somit wieder eine Stellung der Mittel 26 zum Auskoppeln, bei der mehr Energie durchgelassen wird, und eine Stellung, bei der weniger Energie durchgelassen wird. Es ist hierbei unerheblich, ob letztendlich die Strahlprofilanteile 42 und 42' oder die Strahlprofilanteile 44 und 44' ausgekoppelt werden. Aus konstruktiven Gründen wird man dazu neigen, die Situation zu bevorzugen, bei der der Strahlprofilanteil 42 und 42' weiter verwendet wird, d.h. der Hauptlaserstrahl geradlinig weiterläuft.

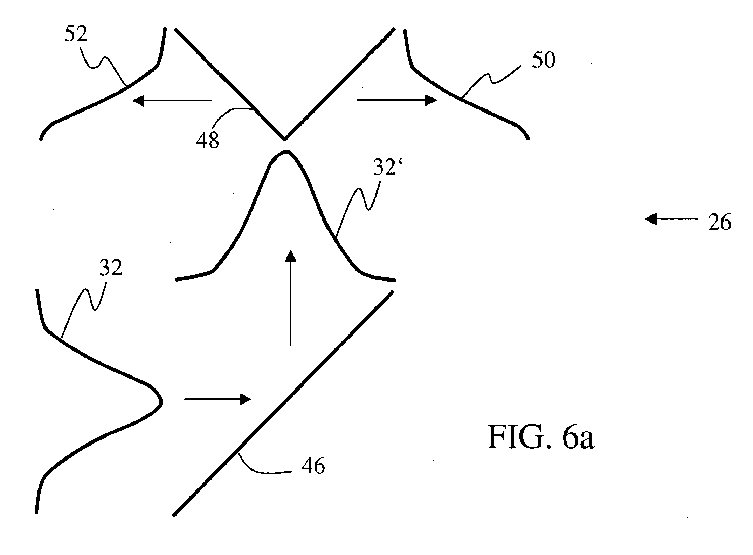

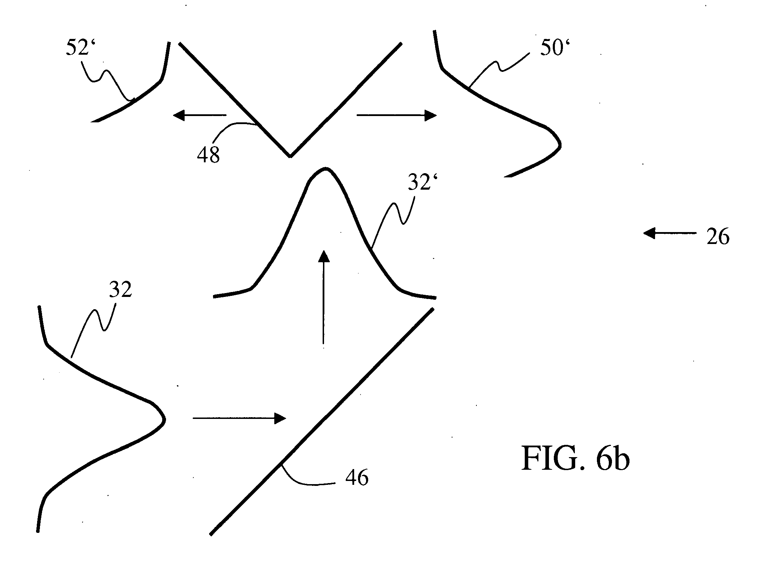

Bei einer weiteren Alternative umfassen die Mittel 26 zum Auskoppeln einen Spiegel 46 und ein optisches Prisma 48. Der Spiegel 46 reflektiert das einfallende Laserstrahlenprofil 32 zum Laserstrahlenprofil 32', und das optische Prisma 48 teilt das Laserstrahlenprofil 32 in zwei Teillaserstrahlen 50 und 52 auf. Das optische Prisma 48 ist versetzbar, vgl. Fig. 6a und 6b. Bei der Stellung gemäß Fig. 6a ist das optische Prisma 48 zentrisch angeordnet, so dass die Teillaserstrahlen 50 und 52 jeweils die Hälfte der Energie des Laserstrahlprofils 32' haben. Bei der Stellung gemäß Fig. 6b ist das optische Prisma 48 derart versetzt, dass der Teillaserstrahl 50' einen größeren Energiegehalt hat als der Teillaserstrahl 52', also mehr als die Hälfte. Auch hier steht somit einmal eine höhere Energie zur Verfügung, zum Beispiel bei dem Teillaserstrahl 50', und einmal eine niedrigere Energie, zum Beispiel bei dem Teillaserstrahl 50.

Allen Ausführungsformen ist gemeinsam, dass die Mittel 26 zum Auskoppeln recht kurzfristig ansteuerbar sind. Durch sie kann der Energiegehalt des letztendlich zu dem Patientenauge 12 gelangenden Laserstrahl schneller geändert werden, als dies mit einer Änderung der Einstellungen bei der Laserstrahlenquelle 14 möglich wäre.

## Patentansprüche

1. Lasersystem (10') zum Ablatieren von Hornhaut an einem Patientenauge (12), mit einer Laserquelle (14), die im Betrieb einen Laserstrahl (16) aussendet, und mit einer Zieleinrichtung (24), die im Betrieb einen solchen Laserstrahl wahlweise auf unterschiedliche Stellen der Hornhaut des Patientenauges (12) lenkt,
wobei der von der Laserquelle (14) ausgesandte Laserstrahl (16) vor dem Erreichen der Zieleinrichtung (24) Auskoppelmittel (26) durchläuft, die im Betrieb einen durch Wahl zwischen zumindest zwei Möglichkeiten festlegbaren Teil des Laserstrahls auskoppeln, so dass dieser Teil des Laserstrahls nicht das Lasersystem (10') in Richtung Patientenauge verlässt, wobei die Wahl zwischen den bestehenden Möglichkeiten der Festlegung des ausgekoppelten Teils des Laserstrahls (16) durch Ansteuerung der Auskoppelmittel (26) mittels einer Steuereinheit (18) erfolgt
**dadurch gekennzeichnet, dass** das Lasersystem (10') eine den Auskoppelmitteln (26) im Strahlengang nachgeordnete Messeinrichtung (30) zur Messung der Energie und/oder der Energiedichte des Laserstrahls (28) umfasst, wobei Messergebnisse der Messeinrichtung der Steuereinheit (18) zugeführt werden.

2. Lasersystem (10') nach Anspruch 1,
**dadurch gekennzeichnet,**
**dass** die Laserquelle (14) Laserpulse vorbestimmter Dauer mit einer vorbestimmten Frequenz abgibt und die Auskoppelmittel (26) während der Pause zwischen zwei Laserpulsen umschaltbar sind.

3. Lasersystem (10') nach einem der vorhergehenden Ansprüche,
**dadurch gekennzeichnet,**
**dass** die Auskoppelmittel (26) eine Blende (34, 34') umfassen.

4. Lasersystem (10') nach Anspruch 3,
**dadurch gekennzeichnet,**
**dass** die Blende (34) zumindest zwei Möglichkeiten von Blendenöffnungen (36, 36') bietet, die sich durch ihre Größe unterscheiden.

5. Lasersystem (10') nach Anspruch 4,
**dadurch gekennzeichnet,**
**dass** die Blende (34) zu dem eintreffenden Laserstrahl so positioniert ist, dass die Blendenöffnung (36, 36') exzentrisch zum Laserstrahl ist.

6. Lasersystem (10') nach einem der Ansprüche 3 bis 5,
**dadurch gekennzeichnet,**
**dass** die Blende (34') zumindest zwei Möglichkeiten von Blendenöffnungen (36") bietet, die sich durch ihr Zentrum voneinander unterscheiden.

7. Lasersystem (10') nach Anspruch 6, bei dem sich Möglichkeiten der Blendenöffnungen (36") mit gleicher Größe durch ihr Zentrum voneinander unterscheiden.

8. Lasersystem (10') nach einem der vorhergehenden Ansprüche,
**dadurch gekennzeichnet,**
**dass** die Auskoppelmittel (26) einen Strahlteiler (40; 46, 48), umfassen, der den eintreffenden Laserstrahl (32) in zwei Teilstrahlen (42, 44; 42' 44'; 50, 52; 50' 52') aufteilt, von denen nur einer (42, 42'; 50, 50') das Lasersystem (10') zum Patientenauge hin verlässt.

9. Lasersystem (10') nach Anspruch 8,
**dadurch gekennzeichnet,**
**dass** der Strahlteiler (40; 46, 48) zumindest zwei Möglichkeiten bietet, welche Energieanteile die Teilstrahlen (40, 42; 40', 42'; 50, 52; 50', 52') relativ zum eintreffenden Laserstrahl (32) haben.

10. Lasersystem (10') nach Anspruch 9,
**dadurch gekennzeichnet,**
**dass** der Strahlteiler einen verkippbaren, halbdurchlässigen Spiegel (40) umfasst.

11. Lasersystem (10') nach Anspruch 9 oder 10,
**dadurch gekennzeichnet,**
**dass** der Strahlteiler ein optisches Prisma (48) umfasst.

## Claims

1. A laser system (10') for ablating cornea on a patient's eye (12), including a laser source (14) emitting a laser beam (16) in operation, and including an aiming means (24) selectively directing such a laser beam to different locations of the cornea of the patient's eye (12) in operation,
wherein the laser beam (16) emitted from the laser source (14) passes coupling-out means (26) before reaching the aiming means (24), which couple out a part of the laser beam determinable by selection between at least two possibilities in operation such that this part of the laser beam does not exit the laser system (10') towards patient's eye,
wherein the selection between the existing possibilities of determining the coupled-out part of the laser beam (16) is effected by driving the coupling-out means (26) by means of a controller (18),
**characterized in that** the laser system (10') includes a measuring means (30) for measuring the energy and/or the energy density of the laser beam (28), which is located downstream of the coupling-out means (26) in the optical path, wherein measurement results of the measuring means are supplied to the controller (18).

2. The laser system (10') according to claim 1,
**characterized in that**
the laser source (14) outputs laser pulses of predetermined period with a predetermined frequency and the coupling-out means (26) are switchable during the interval between two laser pulses.

3. The laser system (10') according to anyone of the preceding claims,
**characterized in that**
the coupling-out means (26) include a diaphragm (34, 34').

4. The laser system (10') according to claim 3,
**characterized in that**
the diaphragm (34) offers at least two possibilities of apertures (36, 36'), which differ in their size.

5. The laser system (10') according to claim 4,
**characterized in that**
the diaphragm (34) is positioned to the incident laser beam such that the aperture (36, 36') is eccentric to the laser beam.

6. The laser system (10') according to any one of claims 3 to 5,
**characterized in that**
the diaphragm (34') offers at least two possibilities of apertures (36"), which differ from each other in their center.

7. The laser system (10') according to claim 6, in which possibilities of the apertures (36") with the same size differ from each other in their center.

8. The laser system (10') according to anyone of the preceding claims,
**characterized in that**
the coupling-out means (26) include a beam splitter (40; 46, 48) splitting the incident laser beam (32) in two partial beams (42, 44; 42', 44'; 50, 52; 50', 52'), only one (42, 42'; 50, 50') of which exits the laser system (10') towards the patient's eye.

9. The laser system (10') according to claim 8,
**characterized in that**
the beam splitter (40; 46, 48) offers at least two possibilities of which energy portions the partial beams (40, 42; 40', 42'; 50, 52; 50', 52') have relative to the incident laser beam (32).

10. The laser system (10') according to claim 9,
**characterized in that** the beam splitter includes a tiltable, semi-transparent mirror (40).

11. The laser system (10') according to claim 9 or 10,
**characterized in that**
the beam splitter comprises an optical prism (48).

## Revendications

1. Système laser (10') destiné à ablater la cornée d'un oeil (12) de patient, comprenant une source laser (14) qui, en fonctionnement, émet un rayon laser (16), et comprenant un dispositif de visée (24) qui, en fonctionnement, dirige un tel rayon laser au choix sur différents endroits de la cornée de l'oeil (12) du patient,
le rayon laser (16) émis par la source laser (14), avant d'atteindre le dispositif de visée (24), traversant des moyens de découplage (26) qui, en fonctionnement, découplent une partie du rayon laser déterminable par sélection entre au moins deux possibilités, de manière à ce que cette partie du rayon laser ne quitte pas le système laser (10') en direction de l'oeil du patient,
la sélection entre les possibilités existantes de détermination de la partie découplée du rayon laser (16) étant réalisée par commande des moyens de découplage (26) au moyen d'une unité de commande (18),
**caractérisé en ce que** le système laser (10') comprend un dispositif de mesure (30) disposé dans le trajet du faisceau en aval des moyens de découplage (26), destiné à mesurer l'énergie et/ou la densité d'énergie du rayon laser (28), des résultats de mesure du dispositif de mesure étant amenés à l'unité de commande (18).

2. Système laser (10') selon la revendication 1,
**caractérisé en ce**
**que** la source laser (14) délivre avec une fréquence prédéterminée des impulsions laser de durée prédéterminée et en ce que les moyens de découplage (26) sont commutables pendant la pause entre deux impulsions laser.

3. Système laser (10') selon l'une quelconque des revendications précédentes,
**caractérisé en ce**
**que** les moyens de découplage (26) comprennent un diaphragme (34, 34').

4. Système laser (10') selon la revendication 3,
**caractérisé en ce**
**que** le diaphragme (34) offre au moins deux possibilités d'ouverture du diaphragme (36, 36'), qui se distinguent par leur taille.

5. Système laser (10') selon la revendication 4,
**caractérisé en ce**
**que** le diaphragme (34) est positionné de telle manière par rapport au rayon laser entrant que l'ouverture du diaphragme (36, 36') est excentrique par rapport au rayon laser.

6. Système laser (10') selon l'une quelconque des revendications 3 à 5,
**caractérisé en ce**
**que** le diaphragme (34') offre au moins deux possibilités d'ouverture (36"), qui se distinguent l'une de l'autre par leur centre.

7. Système laser (10') selon la revendication 6, dans lequel les possibilités des ouvertures du diaphragme (36") de même taille se distinguent les unes des autres par leur centre.

8. Système laser (10') selon l'une quelconque des revendications précédentes,
**caractérisé en ce**
**que** les moyens de découplage (26) comprennent un séparateur de rayon (40 ; 46, 48) qui divise le rayon laser entrant en deux rayons partiels (42, 44 ; 42', 44' ; 50, 52 ; 50', 52'), dont seulement un (42, 42' ; 50, 50') quitte le système laser (10') en direction de l'oeil du patient.

9. Système laser (10') selon la revendication 8,
**caractérisé en ce**
**que** le séparateur de rayon (40 ; 46, 48) offre au moins deux possibilités de savoir quelles fractions d'énergie ont les rayons partiels (40, 42 ; 40',42' ; 50, 52 ; 50', 52') par rapport au rayon laser entrant (32).

10. Système laser (10') selon la revendication 9,
**caractérisé en ce**
**que** le séparateur de rayon comprend un miroir (40) orientable semi-perméable.

11. Système laser (10') selon la revendication 9 ou 10,
**caractérisé en ce**
**que** le séparateur de rayon comprend un prisme optique (48).
